(19) Europäisches Patentamt
European Patent Office
Office européen des brevets

(11) Publication number: **0 271 192 B1**

# (12) EUROPEAN PATENT SPECIFICATION

(45) Date of publication of patent specification: **08.01.92**

(51) Int. Cl.5: **C07D 473/08**, A61K 31/52

(21) Application number: **87309263.9**

(22) Date of filing: **20.10.87**

The file contains technical information submitted after the application was filed and not included in this specification

## (54) Purine histamine H1 antagonists, their preparation and pharmaceutical compositions containing them.

(30) Priority: **21.10.86 US 921553**
**07.05.87 GB 8710843**

(43) Date of publication of application:
**15.06.88 Bulletin 88/24**

(45) Publication of the grant of the patent:
**08.01.92 Bulletin 92/02**

(84) Designated Contracting States:
**AT BE CH DE ES FR GR IT LI LU NL SE**

(56) References cited:
**EP-A- 0 071 738**
**US-A- 4 426 283**

**ARZNEIMITTEL FORSCHUNG DRUG RESEARCH, vol. 34, no. I, January 1984, pags 1-4, Edito Cantor, Aulendorf, DE; K. THIELE et al.: "Neue biologisch aktive Theophyllin-Derivate"**

(73) Proprietor: **AMERICAN HOME PRODUCTS CORPORATION**
**685, Third Avenue**
**New York, New York 10017(US)**

(72) Inventor: **Abou-Gharbia, Magid Abdel Megid**
**2106 Weatherton Drive Brandywood**
**Wilmington Delaware(US)**
Inventor: **Nielsen, Susan Thomson**
**1817 Shipley Road**
**Wilmington New Castle Delaware(US)**
Inventor: **Webb, Michael Byron**
**967 Penn Circle - D102 King of Prussia**
**Montgomery Pennsylvania(US)**

(74) Representative: **Wileman, David Francis Dr. et al**
**c/o Wyeth Laboratories Huntercombe Lane South**
**Taplow Maidenhead Berkshire SL6 OPH(GB)**

**EP 0 271 192 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to histamine $H_1$ antagonists, more specifically to heterocyclic compounds possessing histamine $H_1$ antagonist activity, to processes for preparing them and to pharmaceutical compositions comprising them.

U S-A- 4,426,383 discloses a group of theophylline derivatives which serve as vasodilators useful for increasing blood flow in the treatment of circulatory insufficiency. The compounds are also disclosed to control blood platelet aggregation, act on the central nervous system (psychic energisers), provide anti-histamine, analgesic, anti-asthmatic and hypotensive actions.

EP-A-71738 discloses theophylline derivatives having inter alia histamine-, serotonine-, bradykynine-antagonistic, anti-anaphylactic and $\beta$-adrenergic stimulating effects.

In accordance with this invention there is provided a group of histamine $H_1$ antagonists of the formula:

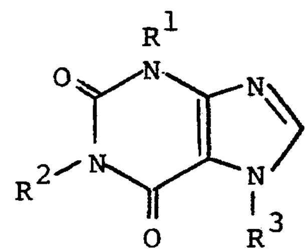

(I)

in which

$R^1$ is hydrogen or alkyl of 1 to 6 carbon atoms; one of $R^2$ and $R^3$ is alkyl of 1 to 6 carbon atoms and the other is

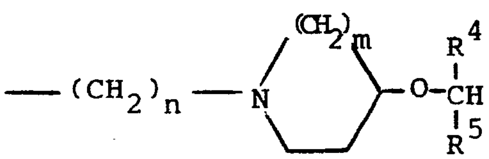

(II)

where

$R^4$ is pyridin-2-yl, pyridin-4-yl, thienyl, or phenyl, any of which is optionally substituted by a halo, alkyl of 1 to 6 carbon atoms, nitro, trifluoromethyl, hydroxy or alkoxy of 1 to 6 carbon atoms substituent;

$R^5$ is pyridin-2-yl, pyridin-4-yl or phenyl, any of which is optionally substituted by a halo, alkyl of 1 to 6 carbon atoms, hydroxy, alkoxy of 1 to 6 carbon atoms, nitro or trifluoromethyl substituent; n is one of the integers from 2 to 10; and m is one of the integers 1, 2 or 3, or a pharmaceutically acceptable salt thereof.

Examples of $R^1$ and $R^2$ are alkyl groups of 1 to 6 carbon atoms. Examples of $R^4$ and $R^5$ are independently phenyl, hydroxyphenyl or halophenyl. Examples of n are 2, 3, 4 or 5. An example of m is 2.

The preferred compounds are those in which $R^1$ is alkyl or 1 to 6 carbon atoms, $R^4$ and $R^5$ are phenyl, hydroxyphenyl or halophenyl (fluorine and chlorine being the most preferred halogens), n is one of integers 2, 3, 4 or 5 and m is 2.

In a preferred embodiment this invention provides a compound of formula Ia:

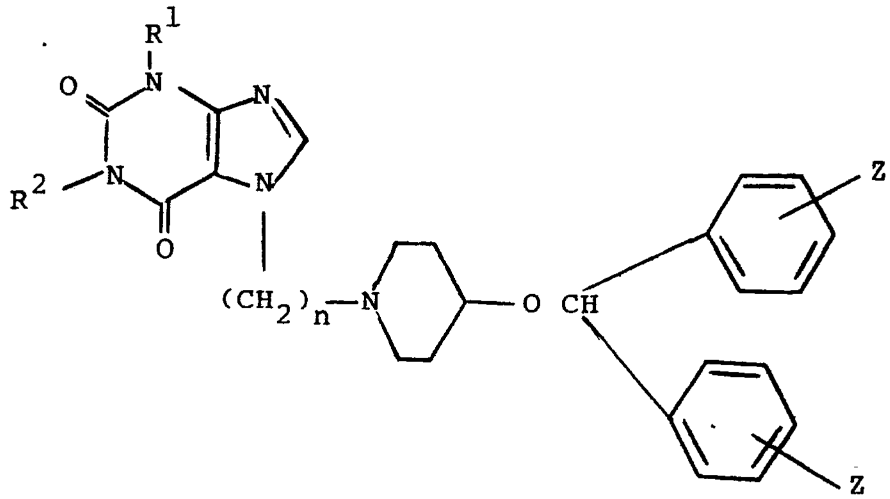

Ia

where

$R^1$ and $R^2$ are alkyl of 1 to 6 carbon atoms, n is one of the integers 2, 3, 4 and 5, and each Z is hydrogen, hydroxy or a halogen, or a pharmaceutically acceptable salt thereof.

The pharmaceutically acceptable salts of the histamine $H_1$ antagonists of this invention are prepared by conventional means with inorganic or organic acids such as hydrochloric, hydrobromic, sulfuric, phosphoric, nitric, acetic, fumaric, citric, tartaric, maleic, lactic, 2-hydroxyethanesulfonic, methanesulfonic, toluene-4-sulfonic or ethanesulfonic acid. An acid addition salt of a compound of formula 1 may be converted to the free base form by standard procedures, e.g. addition of base, such as ammonium hydroxide.

This invention also provides processes for preparing the compounds of the invention. In general the compounds of the invention can be prepared by a variety of synthetic routes using conventional methods. These methods involve coupling appropriate starting materials protected if necessary to form the final compounds.

A first general process for preparing the compounds of formula I comprises reacting a compound of formula

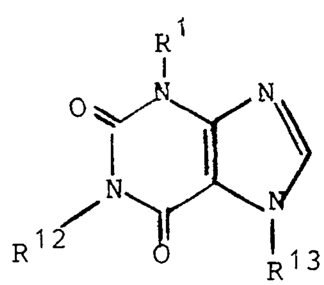

(III)

wherein $R^1$ is as defined hereinabove,
and one of $R^{12}$ and $R^{13}$ is alkyl of 1 to 6 carbon atoms and the other is

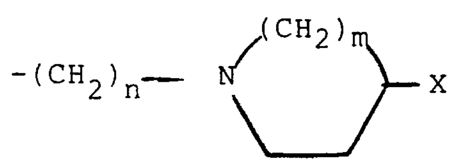

(IIIa)

wherein m and n are as defined hereinabove, with a compound of formula

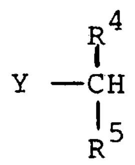

(IV)

wherein $R^4$ and $R^5$ are as defined hereinabove, in which formulae one of X and Y is a halogen or organic sulphonyloxy group, the other is OH or an alkali metal salt thereof.

The aforementioned reaction involves the formation of an ether linkage and may be carried out under conditions known for synthesising such a bond, see for example the Williamson ether synthesis, Merck Index 10th Edn, ONR-96 and references cited therein. Examples of alkali metal salts are the sodium and potassium salts.

The halogen for X and Y is preferably bromine or chlorine. Examples of organic sulphonyloxy groups for X or Y include alkyl-,aryl- or aralkyl-sulphonyloxy groups. Examples of alkylsulphonyloxy groups are those having 1 to 4 carbon atoms, e.g. methanesulphonyloxy. Examples of arylsulphonyloxy groups are those having 6 to 10 carbon atoms, e.g. benzene-and p-toluene-sulphonyloxy groups. The reaction may be conveniently carried out with heating if necessary preferably in a polar solvent, such as dimethylformamide or dimethylsulphoxide. Where an alcohol is used as reactant a base such as an alkali metal carbonate or bicarbonate is preferably present.

Since elimination reactions can occur when X is halogen an organic sulphonyloxy group, it is preferred that X is OH or an alkali metal salt thereof.

A second general process for preparing the compounds of formula I comprises reacting a compound of formula:

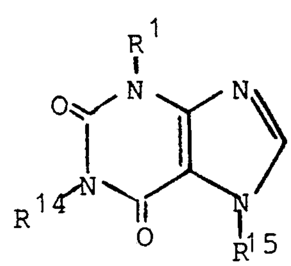

(V)

with a compound of formula

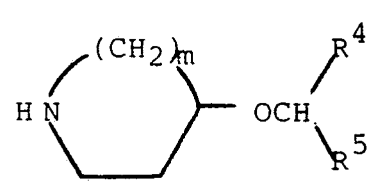

(VI)

in which formula m, $R^1$, $R^4$ and $R^5$ are as defined hereinabove and one of $R^{14}$ and $R^{15}$ is alkyl of 1 to 6 carbon atoms the other is

$-(CH_2)_n-X^1$

wherein n is an integer from 2 to 10 and $X^1$ is halogen or an organic sulphonyloxy radical such as described herein.

The reaction is conveniently carried out in the presence of a base, e.g. triethylamine or potassium carbonate in an inert solvent, e.g. dimethylformamide with heating if necessary.

A third general process for preparing the compound of formula I comprises reacting a compound of formula

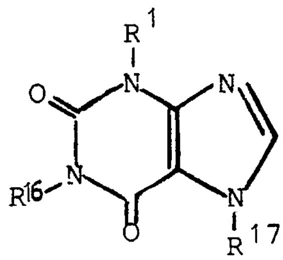

(VII)

with a compound of formula

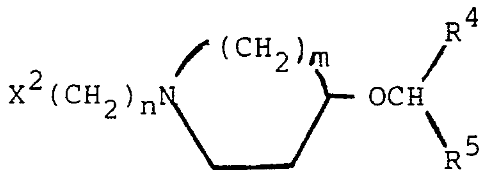

(VIII)

in which formulae $R^1$, n, m, $R^4$ and $R^5$ are as defined above, $X^2$ is halogen or an organic sulphonyloxy group, and one of $R^{16}$ and $R^{17}$ is hydrogen, the other is alkyl of 1 to 6 carbon atoms. This reaction is conveniently carried out in the presence of a base, e.g. triethylamine or sodium hydride, and at room temperature.

In any of the aforementioned processes where a compound of formula I is required wherein $R^1$ is hydrogen, if necessary a protecting group may be employed in the starting material and removed after the reaction to provide the desired compound.

Starting materials used in the aforementioned reactions are either known compounds or can be prepared by analogous or literature methods.

For example the compound of formulae VII wherein $R^1$ and $R^{16}$ are both methyl is theophylline and when $R^1$ and $R^{17}$ are both methyl is theobromine. Other alkyl homologues of theophylline within the scope of formula VII are described in the literature - see for example J H Speer and A L Raymond, J Amer Chem Soc., 75,114(1953) or can be prepared by analogous methods according to the Traube Synthesis (ibid). The following reaction schemes illustrate routes to various starting materials of formula VII:

SCHEME I

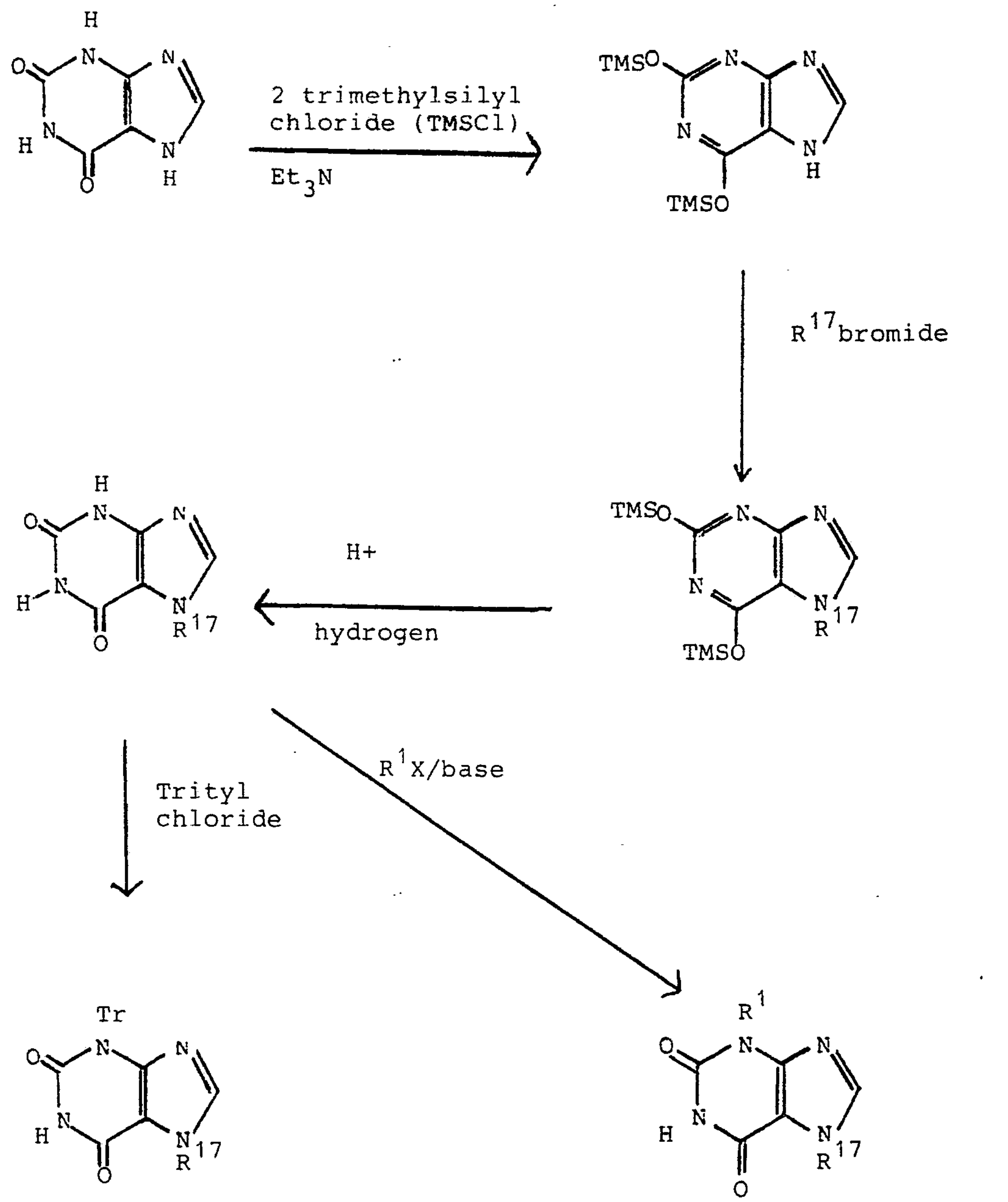


(3-Trityl protected compounds of formula VII: $R^{17}$=alkyl of 1-6 C atoms. Trityl acts as a protecting group so alkylation at the 1 position leads to theobromine type final products where $R^1$ is hydrogen

(VII: $R^1$ and $R^{17}$=alkyl of 1-6 C atoms, $R^{16}$ = hydrogen)

SCHEME II

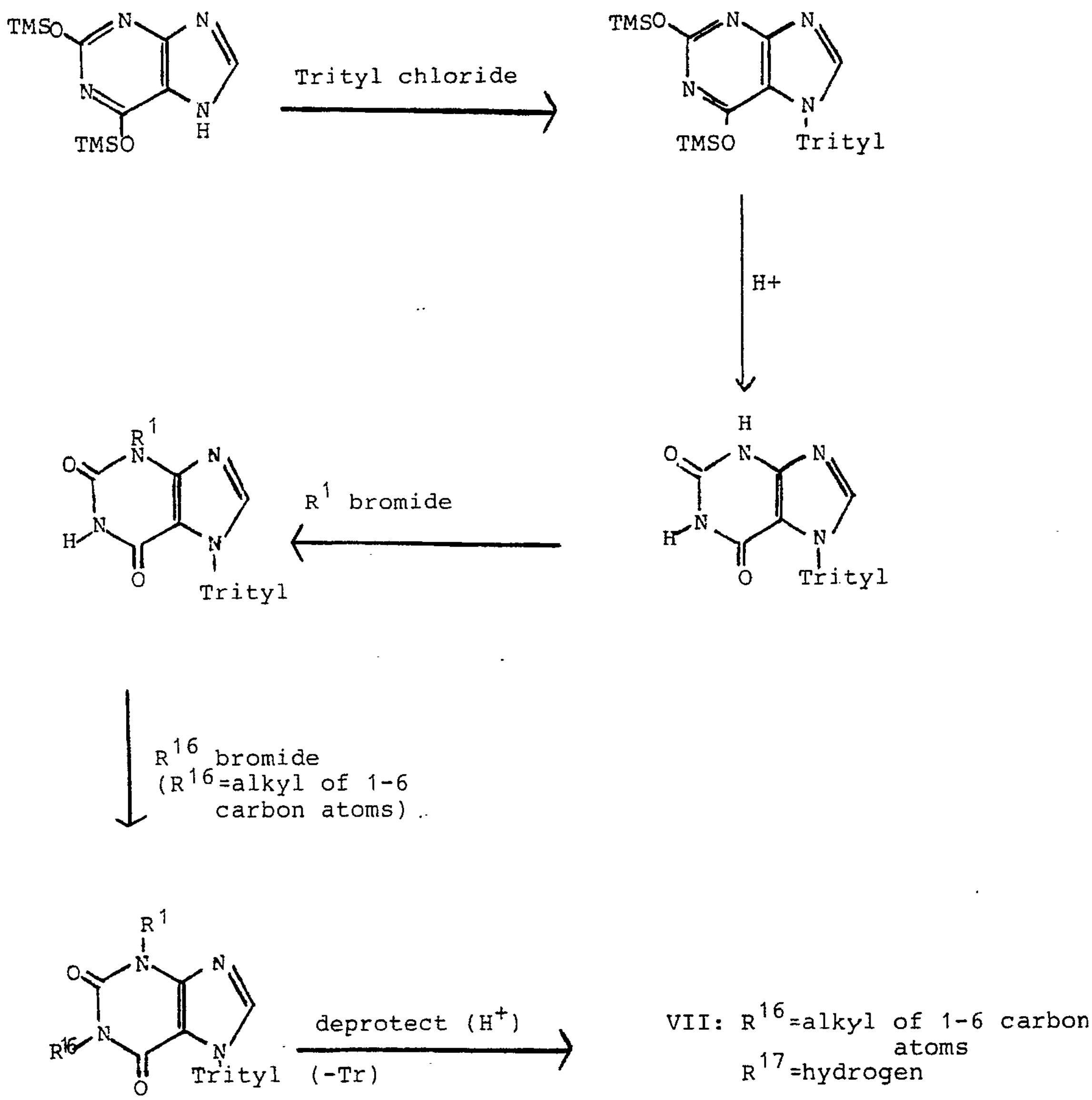
Trityl chloride
H+
R1 bromide
R16 bromide
(R16=alkyl of 1-6
carbon atoms)
deprotect (H+)
(-Tr)
VII: R16=alkyl of 1-6 carbon atoms
R17=hydrogen

SCHEME III

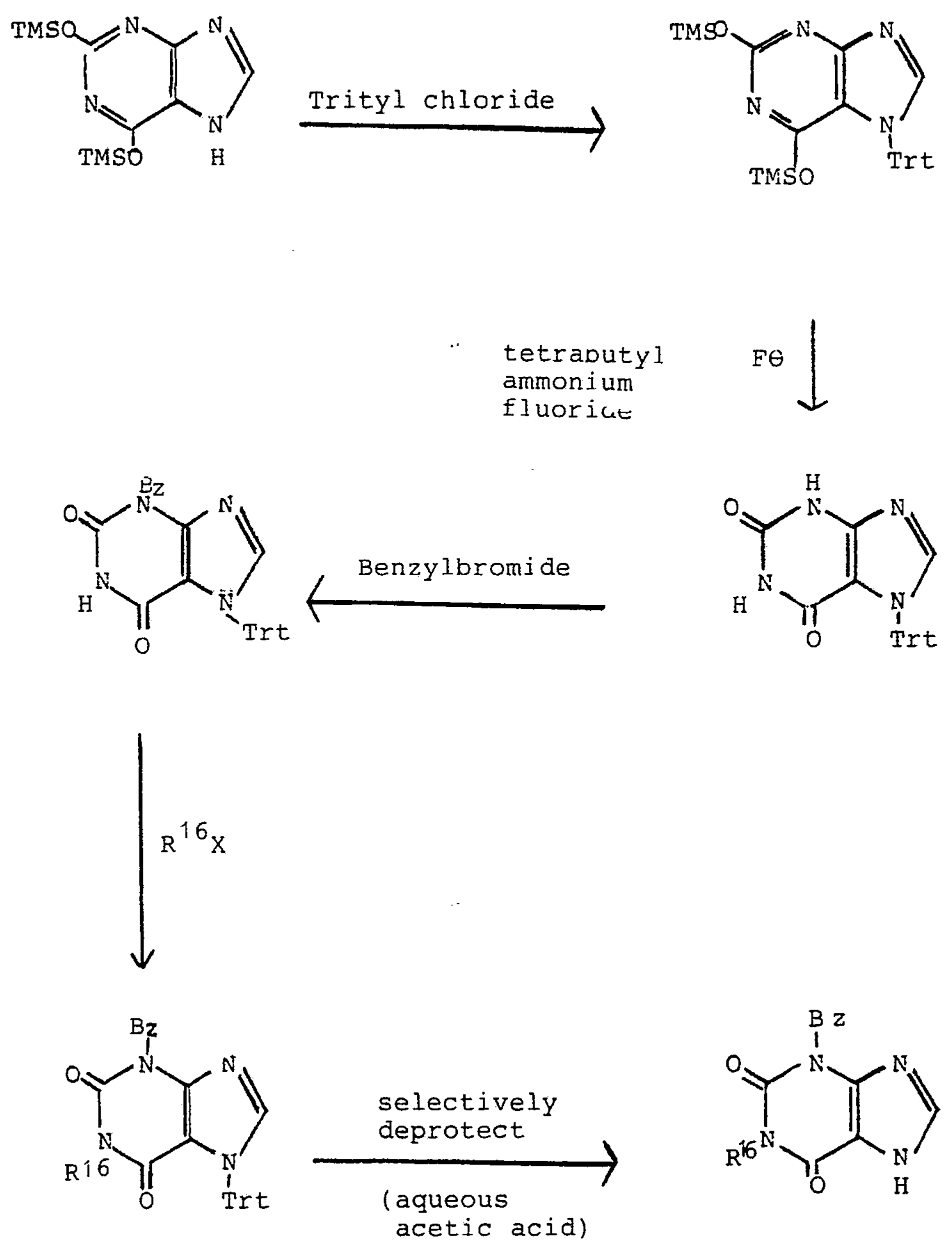


3-benzyl protected compounds of formula VII:$R^{16}$=alkyl of 1-6 carbon atoms. Alkylation occurs at the 7 position leading to theophylline type products.

In reaction Scheme I a route is shown to 3-trityl protected compounds of formula VII, which compounds can be alkylated with a compound of formula VIII as hereinbefore defined and susequently deprotected (aqueous acetic acid) to remove the trityl protecting group and give a compound of formula I wherein $R^1$ is hydrogen and $R^2$ has formula II.

In reaction Scheme III a route is shown to 3-benzyl protected compounds of formula VII which compounds can be alkylated with a compound of formula VIII and subsequently deprotected (hydrogenation) to remove the benzyl group and give a compound of formula I wherein $R^1$ is hydrogen and

$R^3$ has formula II.

Other N-protecting groups known in the art may be used in these reactions and where necessary combinations of such groups may be used which allow selective removal of one, cf Scheme III.

Intermediate compounds of formula III and V may be similarly prepared from appropriate compounds of formula VII initially protected if necessary by alkylating with a compound of the formula

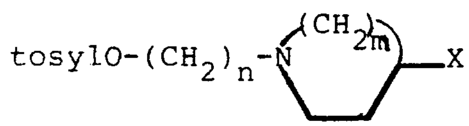

or tosylO-$(CH_2)_n$-$X^1$
in which formulae n, m, X and $X^1$ are as defined above.

Intermediate compounds of formula (VI) may be prepared by reacting a compound of formula

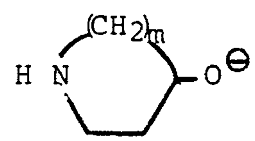

with a compound of formula

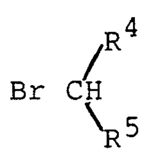

in which formulae m, $R^4$ and $R^5$ are as defined above. Examples of alkali metals are sodium and potassium.

Intermediate compounds of formula VIII are prepared by alkylating a compound of formula VI with a compound of formula.

$X^2$ $(CH_2)_n$O tosyl

wherein $X^2$ and n are as hereinbefore defined.

In a preferred reaction sequence theophylline derivatives in which $R^1$ and $R^2$ are methyl can be prepared by reacting theophylline with a suitable dihalo lower alkane to yield an intermediate product. This intermediate can be reacted with 4-hydroxypiperidine in dimethylformamide in the presence of sodium bicarbonate to afford the unsubstituted piperidinyltheophylline intermediate which then can be reacted with theappropriately substituted bis(phenyl) methyl halide in dimethylformamide in the presence of triethylamine to afford the desired product, thusly:

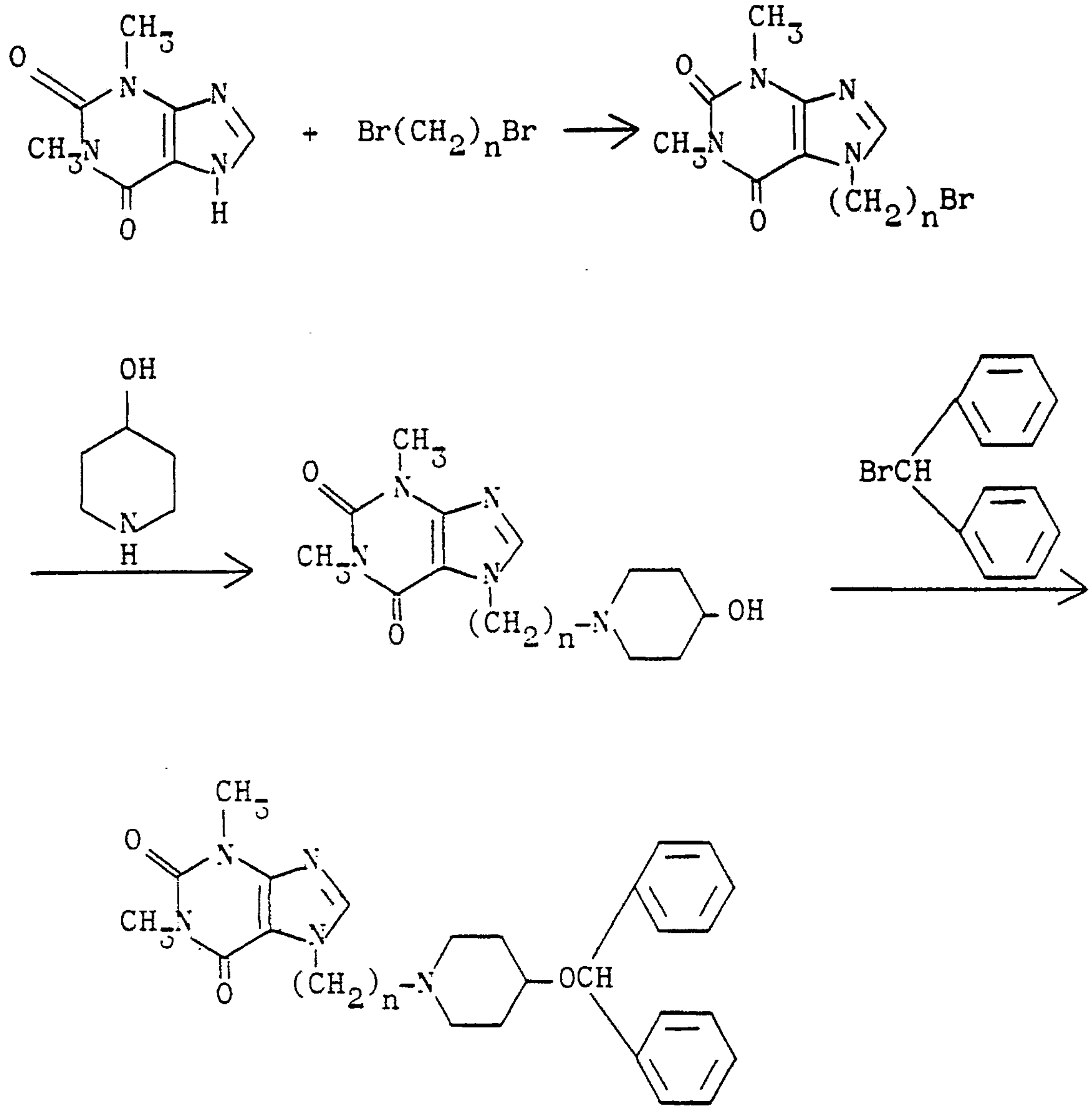

Other starting materials for the processes described herein are known compounds or can be prepared by analogous methods for known compounds.

This invention also provides pharmaceutical compositions comprising a compound of formula I or a pharmaceutically acceptable salt thereof.

For the pharmaceutical compositions any suitable carrier known in the art can be used. In such a composition the carrier may be a solid, liquid or mixture of a solid and a liquid. Solid form compositions include powders, tablets and capsules. A solid carrier can be one or more substances which may also act as flavouring agents, lubricants, solubilisers, suspending agents, binders, or tablet disintegrating agents; it can also be encapsulating material. In powders the carrier is a finely divided solid which is in admixture with the finely divided active ingredient. In tablets the active ingredient is mixed with a carrier having the necessary binding properties and compacted in the shape and size desired. The powders and tablets preferably contain from 5 to 99, preferably 10-80% of the active ingredient.

Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax and cocoa butter. The term 'composition' is intended to include the formulation of an active ingredient with encapsulating material as carrier, to give a capsule in which the active ingredient (with or without other carriers) is surrounded by carriers, which is thus in association with it. Similarly, cachets are included.

Sterile liquid form compositions include sterile solutions, suspension, emulsions, syrups and elixirs.

The active ingredient can be dissolved or suspended in a pharmaceutically acceptable carrier, such as sterile water, sterile organic solvent or a mixture of both. The active ingredient can often be dissolved in a

suitable organic solvent, for instance aqueous propylene glycol containing from 10 to 75% of the glycol by weight is generally suitable. Other compositions can be made by dispersing the finely-divided active ingredient in aqueous starch or sodium carboxymethyl cellulose solution, or in a suitable oil, for instance arachis oil.

Preferably the pharmaceutical composition is in unit dosage form, the composition is sub-divided in unit doses containing appropriate quantities of the active ingredient; the unit dosage form can be a packaged composition, the package containing specific quantities of compositions, for example packeted powders or vials or ampoules. The unit dosage form can be a capsule, cachet or tablet itself, or it can be the appropriate number of any of these in packaged form. The quantity of active ingredient in a unit dose of composition may be varied or adjusted from 10 to 500 mg or more, e.g. 25 mg to 250 mg, according to the particular need and the activity of the active ingredient. The invention also includes the compounds in the absence of carrier where the compounds are in unit dosage form.

The following Examples illustrate the invention and methods for preparing compounds of the invention.

Example 1

7-[3-(4-Diphenylmethoxy)-1-piperidinyl]propyl-3,7-dihydro-1,3-dimethyl-1H-purine-2,6-dione

a) A solution of theophylline (10.0 g, 0.0555 mol), 1,3-dibromopropane (11.6 ml, 23.1 g, 0.114 mol) and triethylamine (10.5 ml, 7.62 g, 0.0753 mol) in N,N-dimethylformamide (250 ml) was stirred at 60° overnight. The stirring was continued one day at room temperature. The solvent was evaporated under reduced pressure. The residue was suspended in $CH_2Cl_2$ and water. An emulsion was caused by a fine solid suspended in the mixture. The solid was filtered out and discarded. The aqueous layer was extracted three times with $CHCl_3$. The combined organic layers were dried with $MgSO_4$. Evaporation of the solvent under reduced pressure gave 7-(3-bromopropyl)-3,7-dihydro-1,3-dimethyl-1H-purine-2,6-dione(12.8g,77% yield).

b) 7-(3-Bromopropyl)-3,7-dihydro-1,3-dimethyl-1H-purine-2,6-dione(4.0 g,0.013 mol), 4-hydroxypiperidine (1.3g, 0.013 mol), and $NaHCO_3$ (2.0g, 0.024 mol) were stirred in dimethylformamide (50ml) at 140° C overnight. The mixture was then refluxed for an additional day. The dimethylformamide was evaporated under reduced pressure. The residue was insoluble in water and $CH_2Cl_2$ and was dissolved in aqueous HCl. The solution was filtered and the separated solid was discarded. The filtrate was basified with aqueous NaOH and the solution was extracted with $CH_2Cl_2$. The extracts were combined and dried with $MgSO_4$. The solvent was evaporated and the residue was purified by HPLC to give 7-[3-(4-hydroxy)-1-piperidinyl]propyl-3,7-dihydro-1,3-dimethyl-1H-purine-2,6-dione(1.3g, 31%yield).

c) 7-[3-(4-Hydroxy-1-piperidinyl)]propyl-3,7-dihydro-1,3-dimethyl-1H-purine-2,6-dione(1.15g, 3.58 mmol) and diphenylmethylbromide (1.80g, 7.28mmol) were added to a suspension of $NaHCO_3$ (0.80 g, 9.5 mmol) in dimethylformamide (60ml) and the reaction mixture was refluxed overnight. The dimethylformamide was evaporated under reduced pressure and the residue was dissolved in $CH_2Cl_2$ and $H_2O$. The aqueous layer was extracted with $CH_2Cl_2$ and the combined $CH_2Cl_2$ extracts were dried with anhydrous $MgSO_4$. The solvent was evaporated under reduced pressure and the residue was purified by HPLC to give the desired product 7-[3-(4-diphenylmethoxy)-1-piperidinyl]propyl-3,7-dihydro-1,3-dimethyl-1H-purine-2,6-dione (0.50g, 29% yield).

d) The fumarate salt was prepared by dissolving the free base in EtOH and adding a solution of fumaric acid in EtOH. The crystalline product was filtered after standing at room temperature for 2 hours to afford the title compound; mp 183-185° C.

Analysis for: $C_{28}H_{33}N_5O_3.C_4H_4O_4.H_2O$

Calculated: C,61.82;H, 6.32;N, 11.27

Found: C, 61.6;H,6.22;N,11.27

Example 2

7-[3-(4-Bis(4-fluorophenyl)methoxy]-1-piperidinyl]propyl-3,7-dihydro-1,3-dimethyl-1H-purine-2,6-dione

The title compound was prepared following the procedure of Example 1 with the exception that bis(4-fluorophenyl)methylbromide was used instead of diphenylmethylbromide. The free base was converted to the monofumarate salt; mp 195-196° C.

Analysis for: $C_{28}H_{31}F_2N_5O_3.C_4H_4O_4$

Calculated: C, 60.08;H,5.52;N,10.95

Found: C,59.95;H,5.67;N.11.81

Example 3

7-[2-(4-Diphenylmethoxy)-1-piperidinyl]ethyl-3,7-dihydro-1,3-dimethyl-1H-purine-2,6-dione

The title compound was prepared following the procedure of Example 1 with the exception that 7-(2-bromoethyl)-3,7-dihydro-1,3-dimethyl-1H-purine-2,6-dione was used instead of 7-(3-bromopropyl)-3,7-dihydro-1,3-dimethyl-1H-purine-2,6-dione. The free base was converted to the monofumarate salt; mp 176-181 °C.
Analysis for: $C_{27}H_{31}N_5O_3.C_4H_4O_4$
Calculated: C,63.09;H,5.98;N,11.88
Found: C, 62.50;H,5.85;N,12.20

Example 4

7-[2-[4-Bis(4-fluorophenyl)methoxy]-1-piperidinyl]ethyl-3,7-dihydro-1,3-dimethyl-1H-purine-2,6-dione

The title compound was prepared following the procedure of Example 3 with the exception that bis(4-fluorophenyl)methylbromide was used instead of diphenylmethylbromide. The free base was converted to the monofumarate salt; mp 191-195 °C.
Analysis for: $C_{27}H_{29}F_2N_5O_3.C_4H_4O_4$.
Calculted: C,59.51;H,5.32;N,11.20
Found: C, 59.46;H,5.49;N.11.20

The compounds of this invention were established to be histamine $H_1$-antagonists by subjecting them to the following standard test procedures for $H_1$-blocking activity:

Fresh segments of terminal ileum immediately proximal to Peyer's patch, obtained from male Buckshire guinea pigs, were suspended in 37 °C Tyrode's solution in a tissue bath and aerated. The tissue segments were placed under one gram tension and allowed to equilibrate for one hour. Histamine was added to each tissue bath to a final concentration of $1 \times 10^{-6}$ M. The contraction response after it equilibrated was noted as grams tension. Test drug was added, in the presence of histamine, to each bath to a final concentration of $1 \times 10^{-7}$ M. The change in grams tension was noted and the percent reduction in grams tension calculated.

Following this procedure, with quadruplicate sets of tissues, the compound of Example 1 demonstrated 92 percent reduction in tissue contraction and the compounds of Examples 2,3 and 4 provided 70,86 and 62 percent reduction in contraction, respectively.

In comparison when a complete histamine dose-response curve was carried out, the ileum tissues then exposed to the compound of Example 1 at a concentration of $10^{-8}$ M and subsequently treated with histamine to repeat the original dose-response curve, a marked, parallel shift of the curve to the right was observed, thus evidencing the requirement for greatly increased histamine concentrations to induce the same tissue contraction. From this study, the histamine $H_1$ antagonist potency of the product of Example 1 was calculated via the formula.

$$K_B = \frac{[\text{Antagonist}]}{[\text{Dose Ratio} - 1]}$$

to equal $2.9 \times 10^{-10}$ M.

In vivo, the compound of Example 1 blocked histamine-induced death in guinea pigs at the doses and after the oral pretreatment times (number of hours prior to histamine administration) shown in the Table.

TABLE

| Compound of Example 1 Dose, mg/kg,p.o. | | Percent Survival | | | | | |
|---|---|---|---|---|---|---|---|
| | | 1 Hour | | 2 Hours | | 18 Hours | |
| | Trial 1 | Trial 2 | Trial 3 | Trial 3 | Trial 4 | Trial 5 | Trial 6 |
| 0.5 | | | | | | 70 | 40 |
| 1.0 | 40 | 10 | 40 | 70 | 70 | 80 | |
| 5.0 | 80 | | | | | | |
| 10.0 | | | | | 60 | | |

From this data it can be seen that the representative compound of this invention produced in Example 1 was orally effective in reducing histamine-induced lethality in guinea pigs the standard experimental animal, at 1, 2 and 18 hours after dosing, with greater efficacy at 18 hours indicating rapid onset of action, with a long duration of action.

The pharmacological results obtained characterize the compounds of this invention as $H_1$-receptor antagonists useful in the treatment of mammals experiencing conditions such as asthma, hay fever, allergic rhinitis, atopic dermatitis, conjunctivis, pruritis, and eczema, or other responses where histamine is released and acts on $H_1$ receptors. As such, they may be administered topically or systemically. Topical administration is advantageously achieved to the skin via creams, ointments or lotions, or via aerosol introduction into the respiratory tract. Systemic administration may be orally, nasally, intrabronchially parentally or rectally. In each instance, conventional formulation amenable to use in the desired administration route is appropriate. Hence, tablets and capsules may be prepared for oral administration, suppositories for rectal administration, isotonic aqueous solutions for intravenous, subcutaneous or intramuscular injection and in aerosol suspensions for inhalation.

As is conventional in the use of antihistamine agents, the appropriate dosage is determined on a subjective basis by initial administration of small amounts, ca. 0.5-15 mg. followed by increasing quantities up to about 400 mg., depending upon the desired route of administration, until the desired symptomatic relief is obtained. The dosage is personalized in this manner for each patient, based upon size, age, type of discomfort, degree of disability, etc., by the physician.

## Claims

**Claims for the following Contracting States : AT, BE, CH, DE, FR, IT, LI, LU, NL, SE**

1. A compound of formula

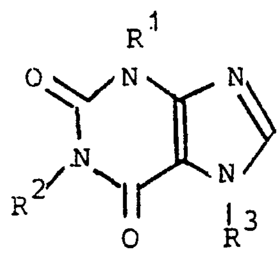

I

in which

$R^1$ is hydrogen or alkyl of 1 to 6 carbon atoms,

one of $R^2$ and $R^3$ is alkyl of 1 to 6 carbon atoms and the other is

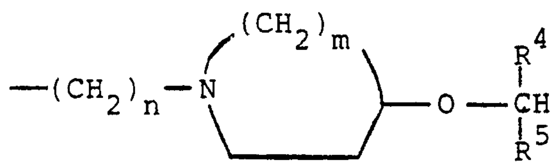

where

$R^4$ is pyridin-2-yl, pyridin-4-yl, thienyl, or phenyl any of which is optionally substituted by a halo, alkyl of 1 to 6 carbon atoms, nitro, trifluoromethyl, hydroxy or alkoxy of 1 to 6 carbon atoms substituent;

$R^5$ is pyridin-2-yl, pyridin-4-yl or phenyl, any of which is optionally substituted by a halo, alkyl of 1 to 6 carbon atoms, hydroxy, alkoxy of 1 to 6 carbon atoms, nitro or trifluoromethyl substituent,

n is one of the integers from 2 to 10 and m is one of the integers 1, 2 or 3 or a pharmaceutically acceptable salt thereof.

2. A compound as claimed in Claim 1 in which $R^1$ is alkyl of 1 to 6 carbon atoms.

3. A compound as claimed in Claim 1 or Claim 2 in which $R^2$ is alkyl of 1 to 6 carbon atoms.

4. A compound as claimed in any one of Claims 1 to 3 in which $R^4$ is phenyl, hydroxphenyl or halophenyl.

5. A compound as claimed in any one of Claims 1 to 4 in which $R^5$ is phenyl, hydroxyphenyl or halophenyl.

6. A compound as claimed in any one of Claims 1 to 5 wherein n is 2, 3, 4 or 5.

7. A compound as claimed in any one of Claims 1 to 6 wherein m is 2.

8. A compound of formula Ia:

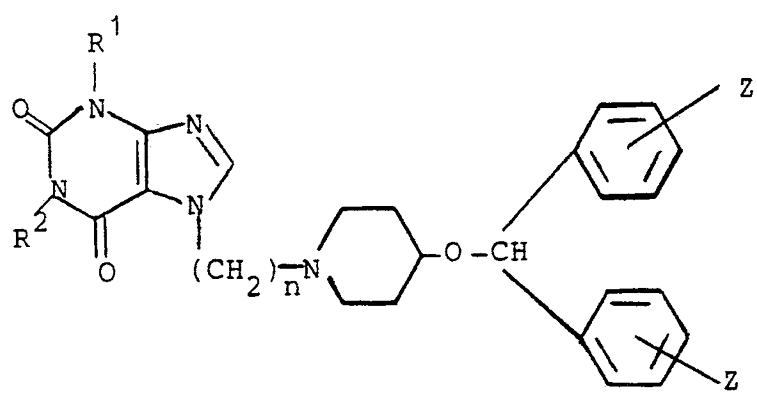

Ia

where

$R^1$ and $R^2$ are alkyl of 1 to 6 carbon atoms, n is one of the integers 2, 3, 4 and 5, and each Z is hydrogen, hydroxy or a halogen, or a pharmaceutically acceptable salt thereof.

9. A compound of formula I which is one of the following 7-[3-(4-Diphenylmethoxy)-1-piperidinyl]propyl-3,7-dihydro-1,3-dimethyl-1H-purine-2,6-dione, 7-[3-[4-Bis(4-fluorophenyl)methoxy]-1-piperidinyl]-propyl-3,7-dihydro-1,3-dimethyl-1H-purine-2,6-dione,7-[2-(4-Diphenylmethoxy)-1-piperidinyl]-ethyl-3,7-dihydro-

1,3-dimethyl-1H-purine-2,6-dione, 7-[2-[4-Bis(4-fluorophenyl) methoxy]-1-piperidinyl]- ethyl-3,7-dihydro-1,3-dimethyl-1H-purine-2,6-dione; or a pharmaceutically acceptable salt thereof.

**10.** A compound as claimed in any one of Claims 1 to 9 when in the form of a salt of an acid selected from hydrochloric, hydrobromic, sulphuric, phosphoric, nitric, acetic, fumaric, citric, tartaric, maleic, lactic, 2-hydroxyethanesulfonic, methanesulfonic, toluene-4-sulfonic and ethanesulfonic acid.

**11.** A process for preparing a compound of formula I or a pharmaceutically acceptable salt thereof, as claimed in Claim 1 which comprises

a) reacting a compound of formula

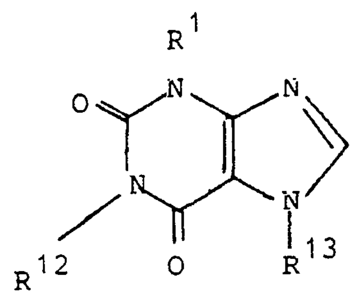

(III)

wherein $R^1$ is as defined in Claim 1 and one of $R^{12}$ and $R^{13}$ is alkyl of 1 to 6 carbon atoms and the other is

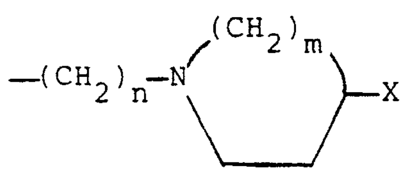

(IIIa)

wherein m and n are as defined in Claim 1, with a compound of formula

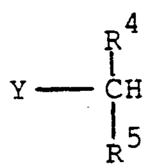

(IV)

wherein $R^4$ and $R^5$ are as defined in claim 1, in which formulae one of X and Y is a halogen or organic sulphonylloxy group, the other is OH or an alkali metal salt thereof,

or

b) reacting a compound of formula:

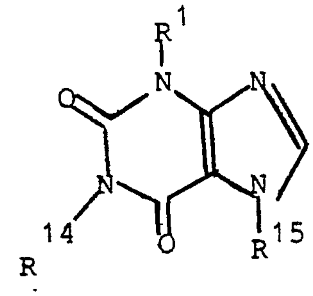

(V)

with a compund of formula

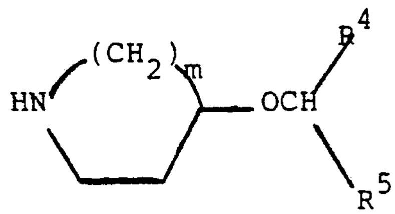

VI

in which formula m, $R^1$, $R^4$ and $R^5$ are as defined in Claim 1 and one of $R^{14}$ and $R^{15}$ is alkyl of 1 to 6 carbon atoms the other is

$-(CH_2)_n-X^1$

wherein n is an integer from 2 to 10 and $X^1$ is halogen or an organic sulphonyloxy radical;
or
c) reacting a compound of formula

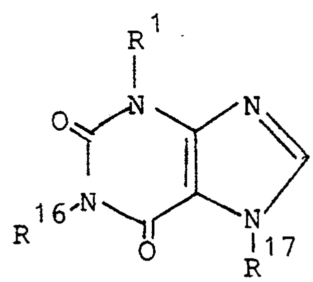

VII

with a compound of formula

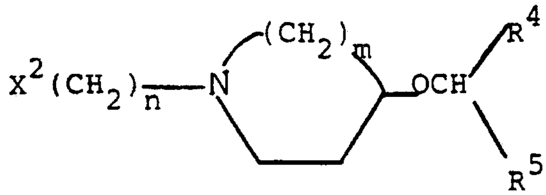

VIII

in which formulae $R^1$, n, m, $R^4$ and $R^5$ are as defined in Claim 1, $X^2$ is halogen or an organic sulphonyloxy group, and one of $R^{16}$ and $R^{17}$ is hydrogen, the other is alkyl of 1 to 6 carbon atoms.
or
d) acidifying a basic compound fo formula I to produce a pharmaceutically acceptable salt thereof or

neutralising a salt of a compound of formula I to give the free base form.

**12.** A modification of process (a), (b) or (c) of Claim 11 in which $R^1$ is a protecting group in the starting material of formula III, V or VII and the protecting group is removed after the reaction to give a compound of formula I wherein $R^1$ is hydrogen.

**13.** A compound of formula I as defined in any one of Claims 1 to 13 or a pharmaceutically acceptable salt thereof for use as a pharmaceutical.

**14.** A pharmaceutical composition comprising a compound of formula I or a pharmaceutically acceptable salt thereof as claimed in any one of Claims 1 to 10 and a pharmaceutically acceptable carrier.

**Claims for the following Contracting States : ES, GR**

**1.** A process for preparing a compound of

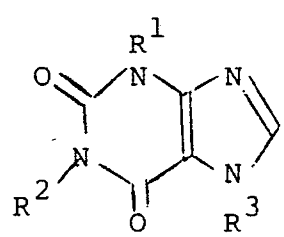

I

in which
$R^1$ is hydrogen or alkyl of 1 to 6 carbon atoms,
one of $R^2$ and $R^3$ is alkyl of 1 to 6 carbon atoms and the other is

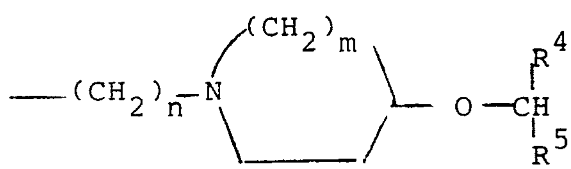

II

where
$R^4$ is pyridin-2-yl, pyridin-4-yl, thienyl, or phenyl any of which is optionally substituted by a halo, alkyl of 1 to 6 carbon atoms, nitro, trifluoromethyl, hydroxy or alkoxy of 1 to 6 carbon atoms substituent;

$R^5$ is pyridin-2-yl, pyridin-4-yl or phenyl, any of which is optionally substituted by a halo, alkyl of 1 to 6 carbon atoms, hydroxy, alkoxy of 1 to 6 carbon atoms, nitro or trifluoromethyl substituent,

n is one of the integers from 2 to 10 and m is one of the integers 1,2 or 3 or a pharmaceutically acceptable salt thereof, which comprises

a) reacting a compound of formula

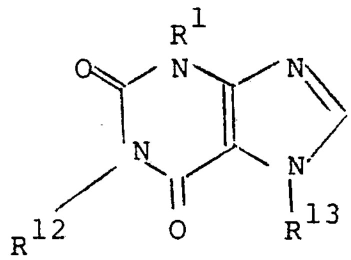

(III)

wherein $R^1$ is as defined above and one of $R^{12}$ and $R^{13}$ is alkyl or 1 to 6 carbon atoms and the other is

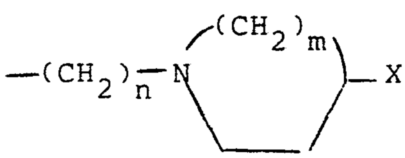

(IIIa)

wherein m and n are as defined above, with a compound of formula

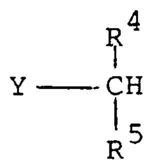

(IV)

wherein $R^4$ and $R^5$ are as defined above, in which formulae one of X and Y is a halogen or organic sulphonyloxy group, the other is OH or an alkali metal salt thereof,
or
b) reacting a compound of formula:

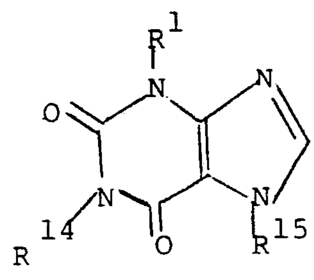

(V)

with a compound of formula

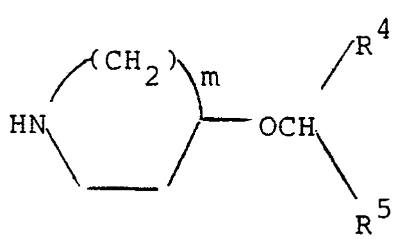

VI

in which formula m, $R^1$, $R^4$ and $R^5$ are as defined above and one of $R^{14}$ and $R^{15}$ is alkyl of 1 to 6 carbon atoms the other is

$-(CH_2)_n-X^1$

wherein n is an integer from 2 to 10 and $X^1$ is halogen or an organic sulphonyloxy radical; or

c) reacting a compound of formula

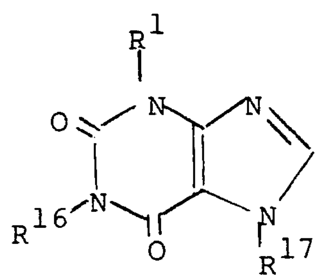

VII

with a compound of formula

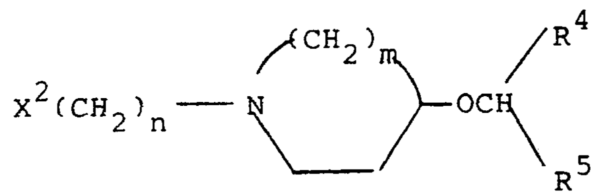

VIII

in which formulae $R^1$, n, m, $R^4$ and $R^5$ are as defined above, $X^2$ is halogen or an organic suphonyloxy group, and one of $R^{16}$ and $R^{17}$ is hydrogen, the other is alkyl of 1 to 6 carbon atoms. or

d) acidifying a basic compound of formula I to produce a pharmaceutically acceptable salt thereof or neutralising a salt of a compound of formula I to give the free base form.

**2.** A modification of process (a), (b) or (c) of Claim 1 in which $R^1$ is a protecting group in the starting material of formula III, V or VII and the protecting group is removed after the reaction to give a compound of formula I wherein $R^1$ is hydrogen.

**3.** A process as claimed in Claim 1 in which $R^1$ is alkyl of 1 to 6 carbon atoms.

**4.** A process as claimed in Claim 1, Claim 2 or Claim 3 in which $R^2$ is alkyl of 1 to 6 carbon atoms.

**5.** A process as claimed in any one of Claims 1 to 4 in which $R^4$ is phenyl, hydroxyphenyl or halophenyl.

**6.** A process as claimed in any one of Claims 1 to 5 in which $R^5$ is phenyl, hydroxyphenyl or halophenyl.

**7.** A process as claimed in any one of Claims 1 to 6 wherein n is 2, 3, 4 or 5.

**8.** A process as claimed in any one of Claims 1 to 7 wherein m is 2.

**9.** A process as claimed in Claim 1 in which the compound prepared has formula Ia:

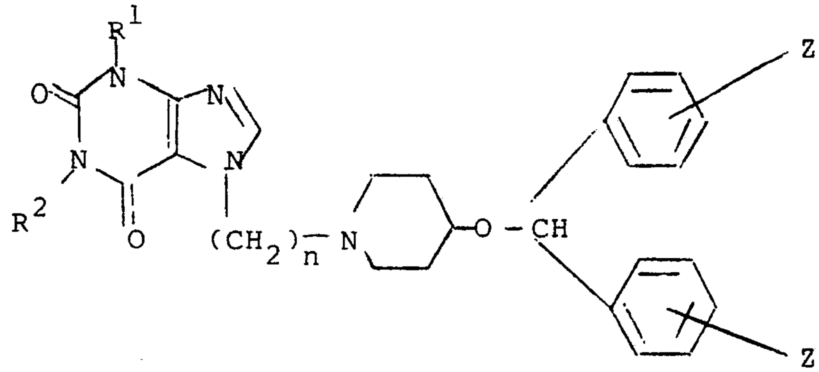

Ia

where
$R^1$ and $R^2$ are alkyl of 1 to 6 carbon atoms, n is one of the integers 2, 3, 4 and 5, and each Z is hydrogen, hydroxy or a halogen, or a pharmaceutically acceptable salt thereof.

**10.** A process as claimed in claim 1 in which the compound prepared is one of the following:

7-[3 (4-diphenylmethoxy)-1-piperidinyl]propyl-3,7-dihydro-1,3-dimethyl-1H-purine-2,6-dione,

7-[3-[4-bis(4-fluorophenyl)methoxy]-1-piperidinyl]propyl-3,7-dihydro-1,3-dimethyl-1H-purine-2,6-dione,

7-[2-(4-diphenylmethoxy)-1-piperidinyl]ethyl-3,7-dihydro-1,3-dimethyl-1H-purine-2,6-dione,

7-[2-[4-bis(4-fluorophenyl)methoxy]-1-piperidinyl]ethyl-3,7-dihydro-1,3-dimethyl-1H-purine-2,6-dione; or a pharmaceutically acceptable salt thereof.

**11.** A process as claimed in any one of Claims 1 to 10 in which the compound prepared is in the form of a salt of an acid selected from hydrochloric, sulphuric, hydrobromic phosphoric, nitric, acetic, fumaric, citric, tartaric, maleic, lactic, 2-hydroxyethanesulfonic, methanesulfonic, toluene-4-sulfonic and ethanesulfonic acid.

**12.** A process for preparing a pharmaceutical composition which comprises bringing a compound of formula I or a pharmaceutically acceptable salt thereof as defined in Claim 1 and a pharmaceutically acceptable carrier into a form suitable for therapeutic administration.

**Revendications**
**Revendications pour les Etats contractants suivants : AT, BE, CH, DE, FR, IT, LI, LU, NL, SE**

**1.** Composé de formule

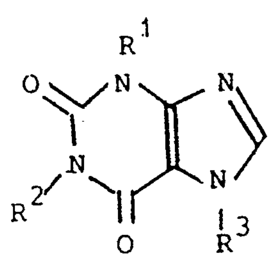

I

dans laquelle

$R^1$ est l'hydrogène ou un groupe alkyle ayant 1 à 6 atomes de carbone, l'un de $R^2$ et $R^3$ est un groupe alkyle ayant 1 à 6 atomes de carbone et l'autre est un groupe

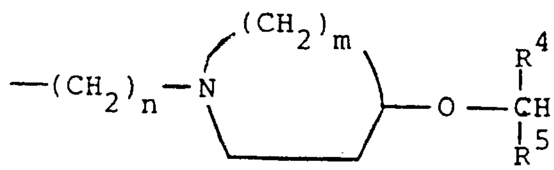

dans lequel

$R^4$ est un groupe pyridine-2-yle, pyridine-4-yle, thiényle, ou phényle, l'un quelconque d'entre eux étant facultativement porteur d'un substituant halogéno, alkyle ayant 1 à 6 atomes de carbone, nitro, trifluorométhyle, hydroxy ou alkoxy ayant 1 à 6 atomes de carbone ;

$R^5$ est un groupe pyridine-2-yle, pyridine-4-yle ou phényle, l'un quelconque d'entre eux étant facultativement porteur d'un substituant halogéno, alkyle ayant 1 à 6 atomes de carbone, hydroxy, alkoxy ayant 1 à 6 atomes de carbone, nitro ou trifluorométhyle,

n est l'un des nombres entiers de 2 à 10 et m est l'un des nombres entiers 1, 2 ou 3, ou un sel pharmaceutiquement acceptable de ce composé.

**2.** Composé suivant la revendication 1, dans lequel $R^1$ est un groupe alkyle ayant 1 à 6 atomes de carbone.

**3.** Composé suivant la revendication 1 ou la revendication 2, dans lequel $R^2$ est un groupe alkyle ayant 1 à 6 atomes de carbone.

**4.** Composé suivant l'une quelconque des revendications 1 à 3, dans lequel $R^4$ est un groupe phényle, hydroxyphényle ou halogénophényle.

**5.** Composé suivant l'une quelconque des revendications 1 à 4, dans lequel $R^5$ est un groupe phényle, hydroxyphényle ou halogénophényle.

**6.** Composé suivant l'une quelconque des revendications 1 à 5, dans lequel n a la valeur 2, 3, 4 ou 5.

**7.** Composé suivant l'une quelconque des revendications 1 à 6, dans lequel m est égal à 2.

**8.** Composé de formule Ia :

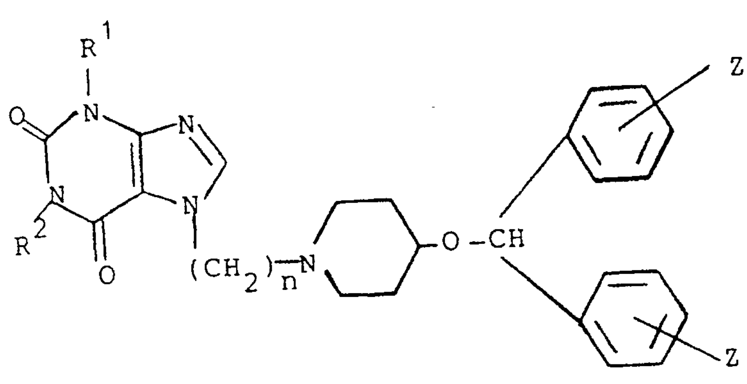

Ia

dans laquelle

$R^1$ et $R^2$ sont des groupes alkyle ayant 1 à 6 atomes de carbone, n est l'un des nombres entiers 2, 3, 4 et 5 et chaque Z représente l'hydrogène, un groupe hydroxy ou un halogène, ou un sel pharmaceutiquement acceptable de ce composé.

**9.** Composé de formule I, qui est l'un des composés suivants : 7-[3-(4-diphénylméthoxy)-1-pipéridinyl]-propyl-3,7-dihydro-1,3-diméthyl-1H-purine-2,6-dione, 7-[3-[4-bis(4-fluoropohényl)méthoxy]-1-pipéridinyl]-propyl-3,7-dihydro-1,3-diméthyl-1H-purine-2,6-dione, 7-[2-(4-diphénylméthoxy)-1-pipéridinyl]-éthyl-3,7-dihydro-1, 3-diméthyl-1H-purine-2,6-dione, 7-[2-[4-bis-(4-fluorophényl)méthoxy]-1-pipéridinyl]-éthyl-3,7-dihydro-1,3-diméthyl-1H-purine-2,6-dione ; ou un sel pharmaceutiquement acceptable de ce composé.

**10.** Composé suivant l'une quelconque des revendications 1 à 9, sous la forme d'un sel d'un acide choisi entre les acides chlorhydrique, bromhydrique, sulfurique, phosphorique, nitrique, acétique, fumarique, citrique, tartrique, maléique, lactique, 2-hydroxyéthanesulfonique, méthanesulfonique, toluène-4-sulfonique et éthanesulfonique.

**11.** Procédé de préparation d'un composé de formule I ou d'un sel pharmaceutiquement acceptable de ce composé suivant la revendication 1, qui consiste

a) à faire réagir un composé de formule

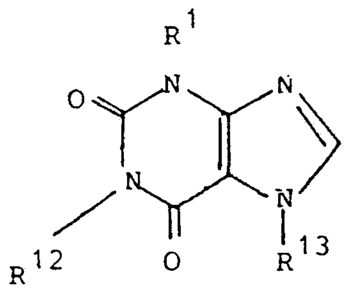

(III)

dans laquelle $R^1$ est tel que défini dans la revendication 1 et l'un de $R^{12}$ et $R^{13}$ est un groupe alkyle ayant 1 à 6 atomes de carbone et l'autre est un groupe

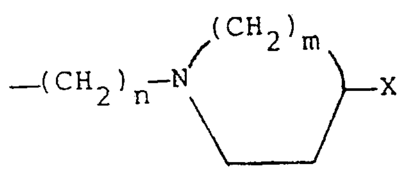

(IIIa)

dans lequel m et n sont tels que définis dans la revendication 1, avec un composé de formule

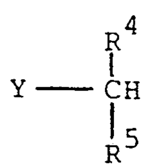

(IV)

dans laquelle $R^4$ et $R^5$ sont tels que définis dans la revendication 1, formules dans lesquelles l'un de X et Y est un halogène ou un groupe organique sulfonyloxy et l'autre est un groupe OH ou un sel de métal alcalin de ce groupe,

ou bien

b) à faire réagir un composé de formule :

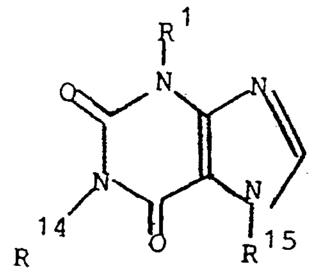

(V)

avec un composé de formule

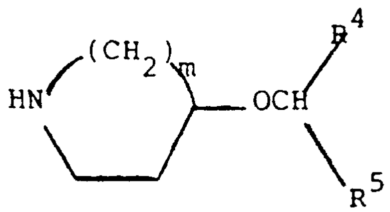

VI

formules dans lesquelles m, $R^1$, $R^4$ et $R^5$ sont tels que définis dans la revendication 1 et l'un de $R^{14}$ et $R^{15}$ est un groupe alkyle ayant 1 à 6 atomes de carbone, l'autre étant un groupe

$-(CH_2)_n-X^1$

dans lequel n est un nombre entier de 2 à 10 et $X^1$ est un halogène ou un radical organique sulfonyloxy ;
ou bien
c) à faire réagir un composé de formule

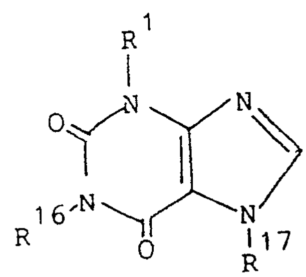

VII

avec un composé de formule

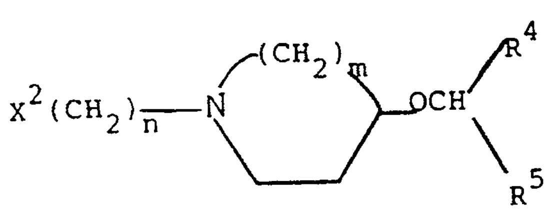

VIII

formules dans lesquelles $R^1$, n, m, $R^4$ et $R^5$ sont tels que définis dans la revendication 1, $X^2$ est un halogène ou un groupe organique sulfonyloxy et l'un de $R^{16}$ et $R^{17}$ est l'hydrogène, l'autre est un

groupe alkyle ayant 1 à 6 atomes de carbone,
ou bien
d) à acidifier un composé basique de formule I pour produire un sel pharmaceutiquement acceptable de ce composé ou à neutraliser un sel d'un composé de formule I pour obtenir la forme base libre.

12. Modification du procédé (a), (b) ou (c) suivant la revendication 11, dans laquelle $R^1$ est un groupe protecteur dans la matière de départ de formule III, V ou VII et le groupe protecteur est éliminé après la réaction en donnant un composé de formule I dans laquelle $R^1$ est l'hydrogène.

13. Composé de formule I suivant l'une quelconque des revendications 1 à 13 ou un sel pharmaceutiquement acceptable de ce composé, destiné à être utilisé comme produit pharmaceutique.

14. Composition pharmaceutique comprenant un composé de formule I ou un sel pharmaceutiquement acceptable de ce composé suivant l'une quelconque des revendications 1 à 10 et un support acceptable du point de vue pharmaceutique.

**Revendications pour les Etats contractants suivants : ES, GR**

1. Procédé de préparation d'un composé de formule

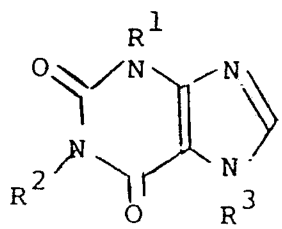

I

dans laquelle

$R^1$ est l'hydrogène ou un groupe alkyle ayant 1 à 6 atomes de carbone, l'un de $R^2$ et $R^3$ est un groupe alkyle ayant 1 à 6 atomes de carbone et l'autre est un groupe

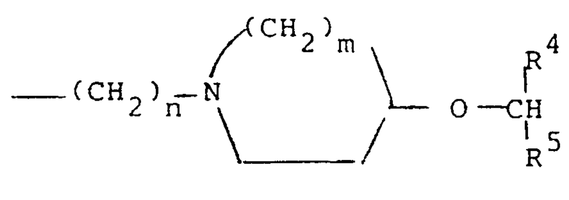

II

dans lequel

$R^4$ est un groupe pyridine-2-yle, pyridine-4-yle, thiényle ou phényle, l'un quelconque d'entre eux étant facultativement porteur d'un substituant halogéno, alkyle ayant 1 à 6 atomes de carbone, nitro, trifluorométhyle, hydroxy ou alkoxy ayant 1 à 6 atomes de carbone ;

$R^5$ est un groupe pyridine-2-yle, pyridine-4-yle ou phényle, l'un quelconque d'entre eux étant facultativement porteur d'un substituant halogéno, alkyle ayant 1 à 6 atomes de carbone, hydroxy, alkoxy ayant 1 à 6 atomes de carbone, nitro ou trifluorométhyle,

n est l'un des nombres entiers de 2 à 10 et m est l'un des nombres entiers 1, 2 ou 3, ou d'un sel pharmaceutiquement acceptable de ce composé, qui consiste

a) à faire réagir un composé de formule

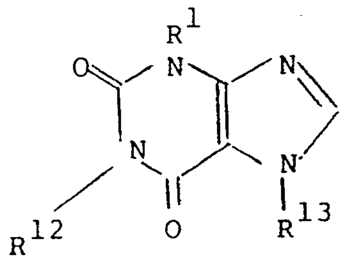

(III)

dans laquelle $R^1$ est tel que défini ci-dessus et l'un de $R^{12}$ et $R^{13}$ est un groupe alkyle ayant 1 à 6 atomes de carbone et l'autre est un groupe

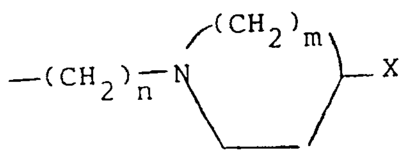

(IIIa)

dans lequel m et n sont tels que définis ci-dessus, avec un composé de formule

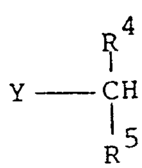

(IV)

dans laquelle $R^4$ et $R^5$ sont tels que définis ci-dessus, formules dans lesquelles l'un de X et Y est un halogène ou un groupe organique sulfonylcxy, l'autre est un groupe OH ou un sel de métal alcalin de ce groupe
ou bien
b) à faire réagir un composé de formule

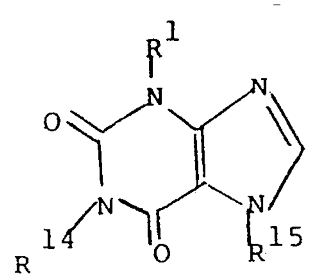

(V)

avec un composé de formule

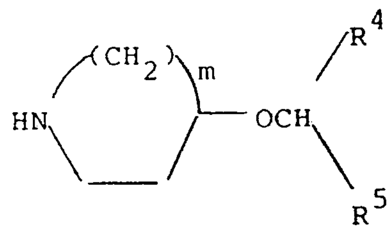

VI

formules dans lesquelles m, $R^1$, $R^4$ et $R^5$ sont tels que définis ci-dessus et l'un de $R^{14}$ et $R^{15}$ est un groupe alkyle ayant 1 à 6 atomes de carbone, l'autre étant un groupe

$-(CH_2)_n-X^1$

dans lequel n est un nombre entier de 2 à 10 et $X^1$ est un halogène ou un radical organique sulfonyloxy ;
ou bien
c) à faire réagir un composé de formule

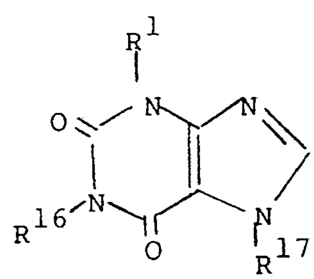

VII

avec un composé de formule

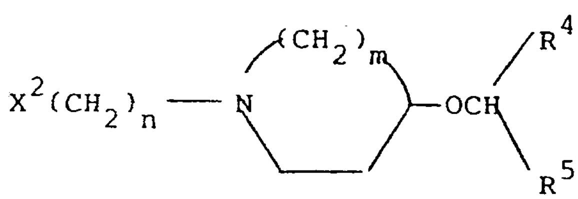

VIII

formules dans lesquelles $R^1$, n, m, $R^4$ et $R^5$ sont tels que définis ci-dessus, $X^2$ est un halogène ou un groupe organique sulfonyloxy et l'un de $R^{16}$ et $R^{17}$ est l'hydrogène, l'autre étant un groupe alkyle ayant 1 à 6 atomes de carbone
ou bien
d) à acidifier un composé basique de formule I pour produire un sel pharmaceutiquement acceptable de ce composé ou à neutraliser un sel d'un composé de formule I pour obtenir la forme base libre.

**2.** Modification du procédé (a), (b) ou (c) suivant la revendication 1, dans laquelle $R^1$ est un groupe protecteur dans la matière de départ de formule III, V ou VII et le groupe protecteur est éliminé après la réaction avec formation d'un composé de formule I dans laquelle $R^1$ est l'hydrogène.

**3.** Procédé suivant la revendication 1, dans lequel $R^1$ est un groupe alkyle ayant 1 à 6 atomes de carbone.

4. Procédé suivant la revendication 1, la revendication 2 ou la revendication 3, dans lequel $R^2$ est un groupe alkyle ayant 1 à 6 atomes de carbone.

5. Procédé suivant l'une quelconque des revendications 1 à 4, dans lequel $R^4$ est un groupe phényle, hydroxyphényle ou halogénophényle.

6. Procédé suivant l'une quelconque des revendications 1 à 5, dans lequel $R^5$ est un groupe phényle, hydroxyphényle ou halogénophényle.

7. Procédé suivant l'une quelconque des revendications 1 à 6, dans lequel n a la valeur 2, 3, 4 ou 5.

8. Procédé suivant l'une quelconque des revendications 1 à 7, dans lequel m est égal à 2.

9. Procédé suivant la revendication 1, dans lequel le composé préparé répond à la formule Ia :

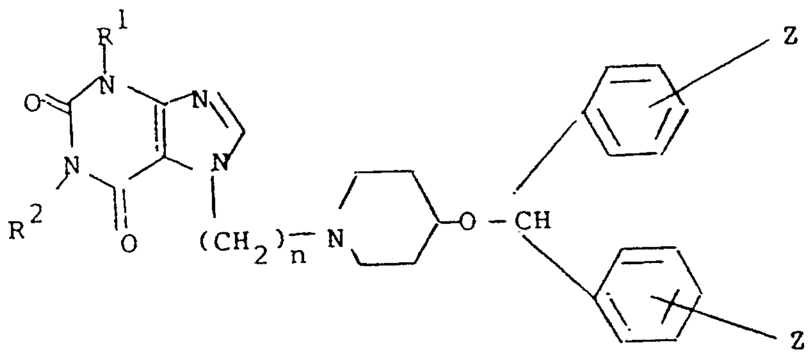

Ia

dans laquelle
$R^1$ et $R^2$ sont des groupes alkyle ayant 1 à 6 atomes de carbone, n est l'un des nombres entiers 2, 3, 4 et 5 et chaque Z est l'hydrogène, un groupe hydroxy ou un halogène, ou un sel pharmaceutiquement acceptable de ce composé.

10. Procédé suivant la revendication 1, dans lequel le composé préparé est l'un des suivants :
7-[3-(4-diphénylméthoxy)-1-pipéridinyl]propyl-3,7-dihydro-1,3-diméthyl-1H-purine-2,6-dione,
7-[3-[4-bis(4-fluoropohényl)méthoxy]-1-pipéridinyl]-propyl-3,7-dihydro-1,3-diméthyl-1H-purine-2,6-dione,
7-[2-(4-diphénylméthoxy)-1-pipéridinyl]-éthyl-3,7-dihydro-1,3-diméthyl-1H-purine-2,6-dione,
7-[2-[4-bis(4-fluorophényl)méthoxy]-1-pipéridinyl]-éthyl-3,7-dihydro-1,3-diméthyl-1H-purine-2,6-dione ;
ou un sel pharmaceutiquement acceptable de ce composé.

11. Procédé suivant l'une quelconque des revendications 1 à 10, dans lequel le composé préparé est sous la forme d'un sel d'un acide choisi entre les acides chlorhydrique, sulfurique, bromhydrique, phosphorique, nitrique, acétique, fumarique, citrique, tartrique, maléique, lactique, 2-hydroxyéthanesulfonique, méthanesulfonique, toluène-4-sulfonique et éthanesulfonique.

12. Procédé de préparation d'une composition pharmaceutique, qui consiste à mettre sous une forme qui convient pour l'administration thérapeutique un composé de formule I ou un sel pharmaceutiquement acceptable de ce composé tel que défini dans la revendication 1 et un support acceptable du point de vue pharmaceutique.

**Patentansprüche**
**Patentansprüche für folgende Vertragsstaaten : AT, BE, CH, DE, FR, IT, LI, LU, NL, SE**

1. Verbindung der Formel

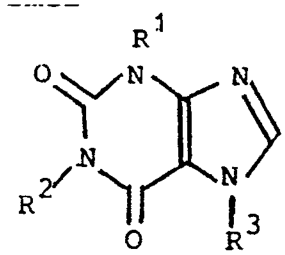

(I),

worin $R^1$ Wasserstoff oder Alkyl mit 1 bis 6 Kohlenstoffatomen bedeutet, eines von $R^2$ und $R^3$ Alkyl mit 1 bis 6 Kohlenstoffatomen darstellt und das andere

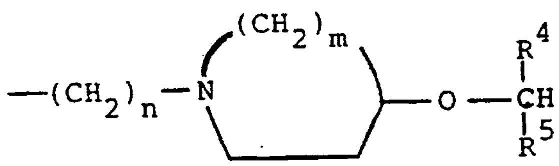

(II)

ist, worin

$R^4$ Pyridin-2-yl, Pyridin-4-yl, Thienyl oder Phenyl bedeutet, die jeweils gegebenenfalls durch einen Halogen-, Alkyl- mit 1 bis 6 Kohlenstoffatomen, Nitro-, Trifluormethyl-, Hydroxy- oder Alkoxysubstituenten mit 1 bis 6 Kohlenstoffatomen substituiert sind;

$R^5$ Pyridin-2-yl, Pyridin-4-yl oder Phenyl darstellt, die jeweils gegebenenfalls durch einen Halogen-, Alkyl- mit 1 bis 6 Kohlenstoffatomen, Hydroxy-, Alkoxy- mit 1 bis 6 Kohlenstoffatomen, Nitro-, oder Trifluormethylsubstituenten substituiert sind;

n eine ganze Zahl von 2 bis 10 und m 1, 2 oder 3 ist, oder ein pharmazeutisch annehmbares Salz hievon.

2. Verbindung nach Anspruch 1, worin $R^1$ ein Alkyl mit 1 bis 6 Kohlenstoffatomen bedeutet.

3. Verbindung nach Anspruch 1 oder 2, worin $R^2$ ein Alkyl mit 1 bis 6 Kohlenstoffatomen bedeutet.

4. Verbindung nach einem der Ansprüche 1 bis 3, worin $R^4$ Phenyl, Hydroxyphenyl oder Halogenphenyl bedeutet.

5. Verbindung nach einem der Ansprüche 1 bis 4, worin $R^5$ Phenyl, Hydroxyphenyl oder Halogenphenyl bedeutet.

6. Verbindung nach einem der Ansprüche 1 bis 5, worin n 2, 3, 4 oder 5 ist.

7. Verbindung nach einem der Ansprüche 1 bis 6, worin m 2 ist.

8. Verbindung der Formel (Ia)

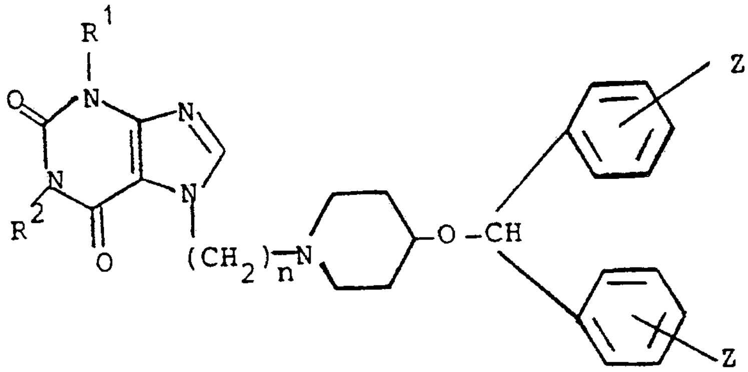

(Ia) ,

worin
$R^1$ und $R^2$ Alkyl mit 1 bis 6 Kohlenstoffatomen bedeuten, n 2, 3, 4 oder 5 ist und jedes Z Wasserstoff, Hydroxy oder ein Halogen darstellt, oder ein pharmazeutisch annehmbares Salz hievon.

**9.** Verbindung der Formel (I), die eine der folgenden: 7-[3-(4-Diphenylmethoxy)-1-piperidinyl]propyl-3,7-dihydro-1,3-dimethyl-1H-purin-2,6-dion, 7-[3-[4-Bis(4-fluorphenyl)methoxy]-1-piperidinyl]-propyl-3,7-dihydro-1,3-dimethyl-1H-purin-2,6-dion, 7-[2-(4-Diphenylmethoxy)-1-piperidinyl]-ethyl-3,7-dihydro-1,3-dimethyl-1H-purin-2,6-dion, 7-[2-[4-Bis(4-fluorphenyl)-methoxy]-1-piperidinyl]-ethyl-3,7-dihydro-1,3-dimethyl-1H-purin-2,6-dion; oder ein pharmazeutisch annehmbares Salz hievon ist.

**10.** Verbindung nach einem der Ansprüche 1 bis 9 in Form eines Salzes einer Säure, ausgewählt aus Chlorwasserstoff-, Bromwasserstoff-, Schwefel-, Phosphor-, Salpeter-, Essig-, Fumar-, Zitronen-, Wein-, Malein-, Milch-, 2-Hydroxyethansulfon-, Methansulfon-, Toluol-4-sulfon- und Ethansulfonsäure.

**11.** Verfahren zur Herstellung einer Verbindung der Formel (I) oder eines pharmazeutisch annehmbaren Salzes nach Anspruch 1, umfassend:
a) Umsetzen einer Verbindung der Formel

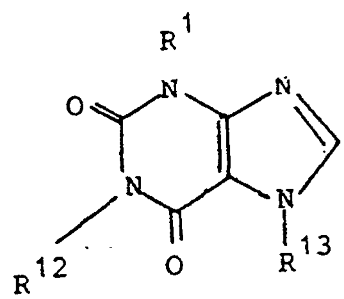

(III),

worin $R^1$ wie in Anspruch 1 definiert ist und eines von $R^{12}$ und $R^{13}$ ein Alkyl mit 1 bis 6 Kohlenstoffatomen darstellt und das andere

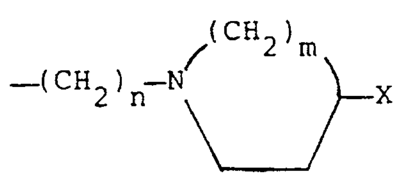

(IIIa)

ist, worin m und n wie in Anspruch 1 definiert sind, mit einer Verbindung der Formel

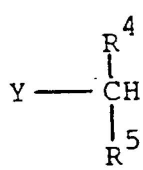

(IV),

worin $R^4$ und $R^5$ wie in Anspruch 1 definiert sind, wobei in den Formeln eines von X und Y ein Halogen oder eine organische Sulfonyloxy-Gruppe darstellt und das andere OH oder ein Alkalimetallsalz hievon bedeutet,
oder
b) Umsetzen einer Verbindung der Formel

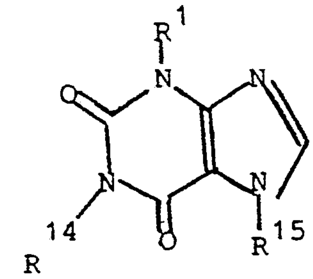

(V)

mit einer Verbindung der Formel

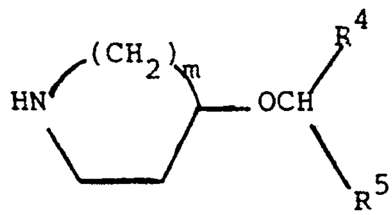

(VI),

wobei in den Formeln m, $R^1$, $R^4$ und $R^5$ wie in Anspruch 1 definiert sind, eines von $R^{14}$ und $R^{15}$ ein Alkyl mit 1 bis 6 Kohlenstoffatomen bedeutet und das andere

$-(CH_2)_n-X^1$

darstellt, worin n eine ganze Zahl von 2 bis 10 ist und $X^1$ Halogen oder einen organischen Sulfonyloxyrest bedeutet;
oder
c) Umsetzen einer Verbindung der Formel

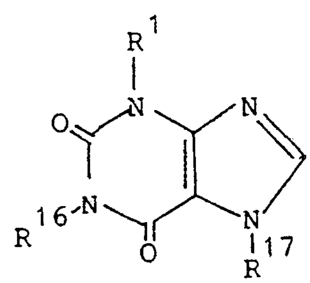

(VII)

mit einer Verbindung der Formel

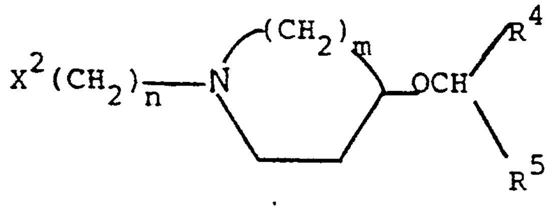

(VIII),

wobei in den Formeln $R^1$, n, m, $R^4$ und $R^5$ wie in Anspruch 1 definiert sind, $X^2$ Halogen oder eine organische Sulfonyloxy-Gruppe bedeutet, eines von $R^{16}$ und $R^{17}$ Wasserstoff bedeutet und das andere ein Alkyl mit 1 bis 6 Kohlenstoffatomen darstellt.
oder

d) Ansäuern einer basischen Verbindung der Formel (I) um ein pharmazeutisch annehmbares Salz hievon herzustellen oder Neutralisieren eines Salzes einer Verbindung der Formel (I), wobei die freie Basenform erhalten wird.

**12.** Abänderung des Verfahrens (a), (b) oder (c) von Anspruch 11, wobei $R^1$ eine Schutzgruppe im Ausgangsmaterial der Formeln (III), (V) oder (VII) ist und die Schutzgruppe nach der Umsetzung entfernt wird, wobei eine Verbindung der Formel (I) erhalten wird, worin $R^1$ Wasserstoff ist.

**13.** Verbindung der Formel (I), wie in einem der Ansprüche 1 bis 12 definiert, oder ein pharmazeutisch annehmbares Salz hievon zur Verwendung als Heilmittel.

**14.** Pharmazeutische Zusammensetzung umfassend eine Verbindung der Formel (I), oder ein pharmazeutisch annehmbares Salz hievon nach einem der Ansprüche 1 bis 10 und einen pharmazeutisch annehmbaren Träger.

**Patentansprüche für folgende Vertragsstaaten : ES, GR**

**1.** Verfahren zur Herstellung einer Verbindung der Formel

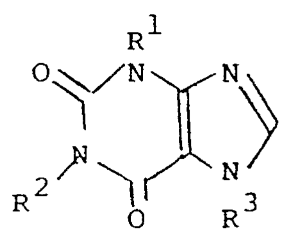

(I),

worin $R^1$ Wasserstoff oder Alkyl mit 1 bis 6 Kohlenstoffatomen bedeutet, eines von $R^2$ und $R^3$ Alkyl mit 1 bis 6 Kohlenstoffatomen darstellt und das andere

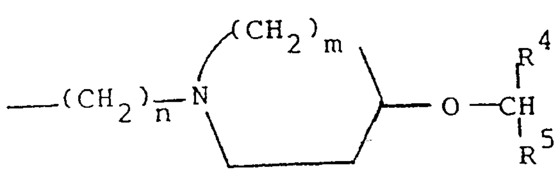

(II)

ist, worin
$R^4$ Pyridin-2-yl, Pyridin-4-yl, Thienyl oder Phenyl bedeutet, die jeweils gegebenenfalls durch einen Halogen-, Alkyl- mit 1 bis 6 Kohlenstoffatomen, Nitro-, Trifluormethyl-, Hydroxy- oder Alkoxysubstituenten mit 1 bis 6 Kohlenstoffatomen substituiert sind;
$R^5$ Pyridin-2-yl, Pyridin-4-yl oder Phenyl darstellt, die jeweils gegebenenfalls durch einen Halogen-, Alkyl- mit 1 bis 6 Kohlenstoffatomen, Hydroxy-, Alkoxy- mit 1 bis 6 Kohlenstoffatomen, Nitro-, oder Trifluormethylsubstituenten substituiert sind;
n eine ganze Zahl von 2 bis 10 und m 1, 2 oder 3 ist, oder eines pharmazeutisch annehmbaren Salzes hievon, umfassend

a) Umsetzen einer Verbindung der Formel

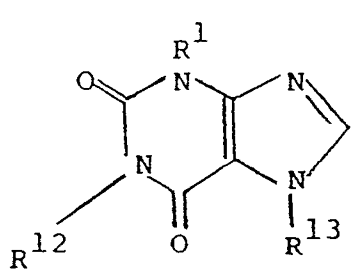

(III),

worin $R^1$ wie obenstehend definiert ist, eines von $R^{12}$ und $R^{13}$ ein Alkyl mit 1 bis 6 Kohlenstoffatomen darstellt und das andere

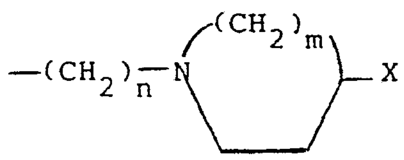

(IIIa)

ist, worin m und n wie obenstehend definiert sind, mit einer Verbindung der Formel

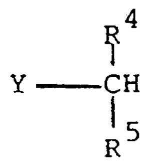

(IV),

worin $R^4$ und $R^5$ wie obenstehend definiert sind, wobei in den Formeln eines von X und Y ein Halogen oder eine organische Sulfonyloxy-Gruppe darstellt und das andere OH oder ein Alkalimetallsalz hievon bedeutet,

oder

b) Umsetzen einer Verbindung der Formel

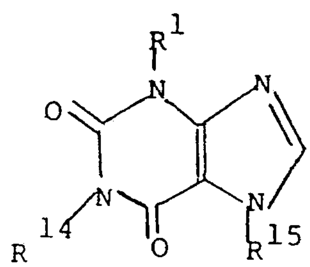

(V),

mit einer Verbindung der Formel

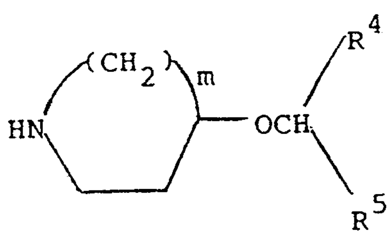

(VI),

wobei in den Formeln m, $R^1$, $R^4$ und $R^5$ wie obenstehend definiert sind, eines von $R^{14}$ und $R^{15}$ ein Alkyl mit 1 bis 6 Kohlenstoffatomen bedeutet und das andere

$-(CH_2)_n-X^1$

darstellt, worin n eine ganze Zahl von 2 bis 10 ist und $X^1$ Halogen oder einen organischen Sulfonyloxyrest bedeutet;

oder

c) Umsetzen einer Verbindung der Formel

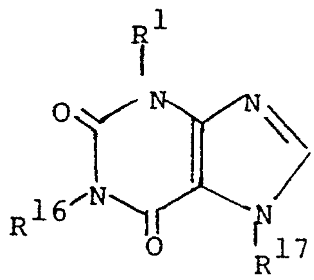

(VII)

mit einer Verbindung der Formel

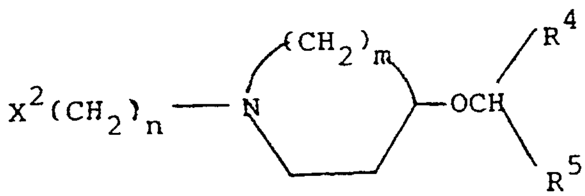

(VIII),

wobei in den Formeln $R^1$, n, m, $R^4$ und $R^5$ wie obenstehend definiert sind, $X^2$ Halogen oder eine organische Sulfonyloxy-Gruppe bedeutet, eines von $R^{16}$ und $R^{17}$ Wasserstoff bedeutet und das andere ein Alkyl mit 1 bis 6 Kohlenstoffatomen darstellt.
oder
d) Ansäuern einer basischen Verbindung der Formel (I) um ein pharmazeutisch annehmbares Salz hievon herzustellen oder Neutralisieren eines Salzes einer Verbindung der Formel (I), wobei die freie Basenform erhalten wird.

**2.** Abänderung des Verfahrens (a), (b) oder (c) von Anspruch 1, wobei $R^1$ eine Schutzgruppe im Ausgangsmaterial der Formeln (III), (V) oder (VII) ist und die Schutzgruppe nach der Umsetzung entfernt wird, wobei eine Verbindung der Formel (I) erhalten wird, worin $R^1$ Wasserstoff ist.

**3.** Verfahren nach Anspruch 1, wobei $R^1$ ein Alkyl mit 1 bis 6 Kohlenstoffatomen bedeutet.

**4.** Verfahren nach Anspruch 1, 2 oder 3, wobei $R^2$ ein Alkyl mit 1 bis 6 Kohlenstoffatomen bedeutet.

**5.** Verfahren nach einem der Ansprüche 1 bis 4, wobei $R^4$ Phenyl, Hydroxyphenyl oder Halogenphenyl bedeutet.

**6.** Verfahren nach einem der Ansprüche 1 bis 5, wobei $R^5$ Phenyl, Hydroxyphenyl oder Halogenphenyl bedeutet.

**7.** Verfahren nach einem der Ansprüche 1 bis 6, wobei n 2, 3, 4 oder 5 ist.

**8.** Verfahren nach einem der Ansprüche 1 bis 7, wobei m 2 ist.

**9.** Verfahren nach Anspruch 1, wobei die hergestellte Verbindung die Formel (Ia) hat,

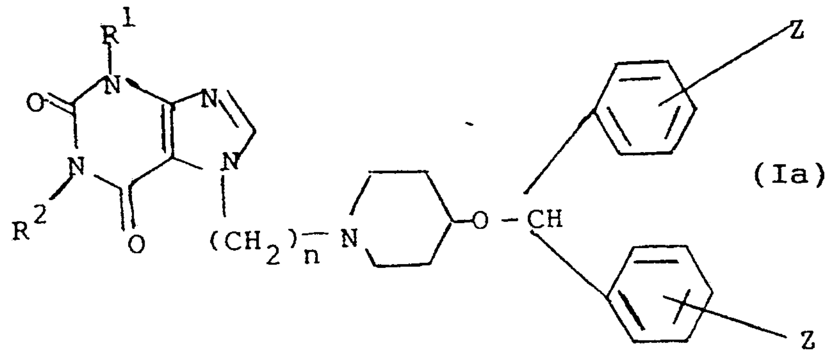

(Ia),

worin

$R^1$ und $R^2$ Alkyl mit 1 bis 6 Kohlenstoffatomen bedeuten, n 2, 3, 4 oder 5 ist und jedes Z Wasserstoff, Hydroxy oder ein Halogen darstellt, oder ein pharmazeutisch annehmbares Salz hievon.

**10.** Verfahren nach Anspruch 1, wobei die hergestellte Verbindung eine der folgenden:
7-[3-(4-Diphenylmethoxy)-1-piperidinyl]propyl-3,7-dihydro-1,3-dimethyl-1H-purin-2,6-dion,
7-[3-[4-Bis(4-fluorphenyl)methoxy]-1-piperidinyl]-propyl-3,7-dihydro-1,3-dimethyl-1H-purin-2,6-dion,
7-[2-(4-Diphenylmethoxy)-1-piperidinyl]-ethyl-3,7-dihydro-1,3-dimethyl-1H-purin-2,6-dion,
7-[2-[4-Bis(4-fluorphenyl)methoxy]-1-piperidinyl]-ethyl-3,7-dihydro-1,3-dimethyl-1H-purin-2,6-dion; oder ein pharmazeutisch annehmbares Salz hievon ist.

**11.** Verfahren nach einem der Ansprüche 1 bis 10, wobei die hergestellte Verbindung in Form eines Salzes einer Säure, ausgewählt aus Chlorwasserstoff-, Schwefel-, Bromwasserstoff-, Phosphor-, Salpeter-, Essig-, Fumar-, Zitronen-, Wein-, Malein-, Milch-, 2-Hydroxyethansulfon-, Methansulfon-, Toluol-4-sulfon- und Ethansulfonsäure, ist.

**12.** Verfahren zur Herstellung einer pharmazeutische Zusammensetzung, umfassend eine Verbindung der Formel (I) oder ein pharmazeutisch annehmbares Salz hievon, wie in Anspruch 1 definiert, und einen pharmazeutisch annehmbaren Träger in eine zur therapeutischen Verabreichung geeignete Form zu bringen.